# EUROPEAN PATENT APPLICATION

(11) **EP 4 471 050 A1**
(43) Date of publication of application: **04.12.2024**
(21) Application number: 23746128.0
(22) Date of filing: 17.01.2023
(51) Int. Cl.: C07K 7/06, A61K 47/60, A61K 38/08, A61P 9/10

(54) **POLYETHYLENE GLYCOL-MODIFIED FORM OF KININ OR DERIVATIVE THEREOF AND PHARMACEUTICAL USE THEREOF**

(30) Priority: 30.01.2022 CN 202210113869
(71) Applicant: Zonhon Biopharma Institute, Inc., Changzhou, Jiangsu 213125 (CN)
(72) Inventor: MA, Bruce Yong, hangzhou, Jiangsu 213125 (CN); ZHUANG, Yu, hangzhou, Jiangsu 213125 (CN); WANG, Jun, hangzhou, Jiangsu 213125 (CN); WANG, He, hangzhou, Jiangsu 213125 (CN); JIANG, Chenyang, hangzhou, Jiangsu 213125 (CN); XU, Zhen, hangzhou, Jiangsu 213125 (CN)
(74) Representative: Axe PI
(86) International application number: PCT/CN2023/072604
(87) International publication number: WO 2023/143246

(57) **Abstract**

The present invention relates to polyethylene glycol-modified form of kinin or derivative thereof and pharmaceutical use thereof. In one aspect, by PEG modification technology, the half-life of the kinin is greatly prolonged, meanwhile, the biological activity of the kinin is exerted, and the problem that pure kinin has extremely short half-life and therefore has no druggability is solved. PEG modifiers used include, but are not limited to, SPA, SCM and SS modifiers, and can be linear or branched PEG modifiers. In another aspect, the kinin variant has obvious advantages, such as the half-life is further prolonged while the affinity activity with receptor is maintained, and the *in vivo* efficacy of the PEG-modified kinin variant is superior to that of PEG-modified wild-type kinin, so that the druggability of the kinin is further improved. In addition, the PEG-modified kinin show remarkable efficacy in various administration routes, especially in subcutaneous injection and oral administration, which is convenient in clinical use, and effectively improves the compliance of a patient, and is more suitable for long-term therapeutic administration, such as the convalescent treatment, relapse prevention and the like of a patient with cerebral stroke.

## Description

### TECHNICAL FIELD

The present invention relates to polyethylene glycol-modified form of kinin or derivative thereof and pharmaceutical use thereof. In particular, PEG modification technology is used to optimize the druggability of kinins.

### DESCRIPTION OF RELATED ART

The Kinin-kallikrein system (KKS system) is an important regulatory system of the body, which participates in a variety of physiological and pathological processes, such as the regulation of cardiovascular, renal and nervous system functions. KKS system includes kallikrein (KLK), kininogen, kinin, kinin receptor (B 1, B2 receptor) and kallikrein, of which kallikrein and kinin are the core components of KKS. At present, it is believed that human tissue kallikrein is composed of at least 15 members (KLK1-KLK15), among which there are many studies on tissue kallikrein 1(KLK1). KLK1 plays a series of biological roles by converting kininogen into kinin and acting on the corresponding receptors. Kinins are a kind of active peptides with very little amount in human body. The well studied kinins include lysyl bradykinin (Lys-BK), bradykinin (BK) and angiotensin.

Lysyl bradykinin is mainly produced by the hydrolysis of LMWK, which is composed of 10 amino acids with the sequence of KRPPGFSPFR. Lysyl bradykinin can be cleaved to bradykinin (9-peptide: RPPGFSPFR) by aminopeptidase *in vivo.* Both of them have a variety of physiological functions such as regulating blood pressure and inflammatory response. They bind to G protein-coupled receptors, activate the phosphorylation of extracellular regulated protein kinases 1/2 (ERK1/2) and cAMP-response element binding protein (CREB), stimulate the release of second messengers such as nitric oxide, cAMP and prostacycline I2, antagonize the action of renin-angiotensin system, thus exerting biological effects, dilating arterioles, increasing local blood flow, increasing vascular permeability and vasodilation.

The degradation of lysyl bradykinin in the body is mainly involved in aminopeptidase, carboxypeptidase and angiotensin ACEII. The dynamic balance of bradykinin in the body is realized based on kallikrein and degrading enzyme system. Exogenous administration of lysyl bradykinin or bradykinin can cause vasodilation, which has the potential to treat cardiovascular and cerebrovascular diseases. However, the half-life of lysyl bradykinin and bradykinin *in vivo* is short, only a few seconds, and they are quickly inactivated by kallikrein hydrolysis, which greatly limits their drug druggability.

At present, the commonly used long-acting strategies of polypeptide drugs include chemical modification, mainly including polyethylene glycol modification, peptide backbone end modification, side chain modification, cyclization, amino acid substitution and glycosylation modification; microspheres embedding method, namely micro-nanoparticle embedding method, includes lipid carrier nanoparticles, polymer carrier nanoparticles and inorganic carrier nanoparticles; protein fusion technology, at present, the commonly used fusion proteins are albumin and immunoglobulin (IgG). At present, various methods have been used, and each has its own advantages and disadvantages. The literature also points out that the micro-nanoparticles embedding method has more potential. (Cheng Nian, Hu Zhongping et al. Research progress of long-acting peptide drugs [J]. Chinese Journal of New Drugs, 2016 (25, 22))

Removal of restriction sites by amino acid substitution to protect peptides from degradation is an effective way to prolong the half-life *in vivo.* Lobradimil can prolong the half-life *in vivo* by mutating Pro at positon 3, Phe at positon 5, Phe at positon 8 and Arg at positon 9 to unnatural amino acids. Its main role is to increase the permeability of the blood-brain barrier. And the drug was safe to treat brain tumors in children when combined with carboplatin, however, the efficacy did not reach the end point (Cancer Chemother Pharmacol. 2006, 58:343-347), the project was discontinued after the clinical phase II. Icatibant, which was approved by FDA in 2011, is also based on bradykinin molecule. Pro at positon 3, Phe at positon 5, Pro at positon 7, and Phe at positon 8 of BK were mutated to unnatural amino acids to prolong its half-life *in vivo.* The indication is hereditary angioedema, and the drug molecule is selective competitive inhibitor of the B2 receptor, acting in contrast to bradykinin. At present, there is no report that amino acid mutation can prolong the half-life of LBK/BK *in vivo* and exert vasodilator biological effects and be successfully used in clinical treatment.

Polyethylene glycol (PEG) modified protein is a way to prolong the half-life *in vivo* and improve the stability. At present, more than ten PEGylated protein drugs have been marketed. However, PEG-modified peptide drugs are rare, because peptides often play their biological functions by binding to receptors *in vivo.* Due to the small size of peptides, PEG modification is likely to affect the binding of peptides to receptors and cause the loss of biological activity. Taking bradykinin as an example, it is only composed of about 9 amino acids and has a small molecular structure. Pegylated lysyl bradykinin or bradykinin has not been reported at home and abroad.

### SUMMARY

The technical problem solved by this application is to prolong the half-life of lysyl bradykinin or bradykinin, while exerting its biological activity *in vivo* and solving its druggability.

On the one hand, the present application significantly extends the half-life of lysyl bradykinin or bradykinin while exerting its *in vivo* biological activity by PEG modification technology.

In the polyethylene glycol-modified lysyl bradykinin or bradykinin of the present invention, the PEG modification is N-terminal modification, but not C-terminal modification. The lysyl bradykinin or bradykinin is wild-type, or derivatives from wild-type lysyl bradykinin or bradykinin (no polyethylene glycol modification). Such derivatives include not only mutants enumerated in the embodiments, but also peptides, fusion proteins (including but not limited to albumin fusion, Fc fusion, etc.), various modification product (except polyethylene glycol modification) which are further modified on the basis of the Lys-BK/BK mutant of this application. Such derivatives retain the activity (binding activity to receptors) of wild-type lysyl bradykinin or bradykinin *in vitro.* The examples show that the stability of PEG modified lysyl bradykinin or bradykinin or its variants is good. And when the variants retain activity *in vitro,* polyethylene glycol modified variants could prolong the half-life and exert *in vivo* biological activity. In contrast, when the variants do not retain *in vitro* activity, the variants or polyethylene glycol modified variants could not exert *in vivo* biological activity. Therefore those skilled in the art could reasonably expect that after modified with polyethylene glycol, other lysyl bradykinin or bradykinin derivatives retaining receptor binding activity will prolong the half-life, and exert *in vivo* biological activity, and not limited to the specific variants as described in the examples.

PEG modifiers include but are not limited to SPA, SCM and SS modifiers. It can be straight-chain or branched PEG modifier.

As preferred, the PEG modifier is straight-chain PEG modifier.

As preferred, the molecular weight of PEG modifier is 2KD-20KD.

As preferred, the molecular weight of PEG modifier is 2KD-10KD.

As preferred, the molecular weight of PEG modifier is 2KD or 10KD.

On the other hand, the druggability is further improved by optimizing the amino acid sequence of lysyl bradykinin or bradykinin.

The present application provides derivative of lysyl bradykinin or bradykinin that introduces cysteine (Cys) at any position of the wild-type lysyl bradykinin or bradykinin. After the introduction of cysteine, it showed significant advantages in drug efficacy *in vivo.* Preferably, the first Lys of wild-type lysyl bradykinin is mutated to Cys, which is CRPPGFSPFR.

The application also provides derivative of lysyl bradykinin or bradykinin in which any number and any kind of amino acids are inserted between the first and second amino acid residues at the N-terminus of wild-type lysyl bradykinin, that is KXRPPGFSPFR, where X denotes any number and any kind of amino acids. An arbitrary amino acid is inserted before the first amino acid residue at the N-terminus of wild-type bradykinin, that is XRPPGFSPFR, where X represents any number and any kind of amino acids. The experimental results showed that the activity was better after prolongation of the N terminus. Preferably, one or two or three amino acids are inserted. Preferably, one or two arginines (Arg) are inserted.

The application also provides derivative of lysyl bradykinin or bradykinin, which is composed of two or more lysyl bradykinin monomers in series (e.g., m14), or composed of two or more bradykinin monomers in series, or composed of both lysyl bradykinin monomers and bradykinin monomers in series (e.g., m12). The lysyl bradykinin or bradykinin monomer may be wild-type lysyl bradykinin or bradykinin, or may be variant after substitution, insertion, or deletion of some amino acids in the wild-type lysyl bradykinin or bradykinin.

The application also provides derivative of lysyl bradykinin or bradykinin in which Phe at positon 6 of wild-type lysyl bradykinin or Phe at positon 5 of wild-type bradykinin is mutated to other amino acids, such as the unnatural amino acids Igl(α-2-indolyl glycine), THI(β-2-thiophenylalanine). LBK or BK mutated at the above sites is not easily recognized by enzymes *in vivo,* thus effectively extending its half-life *in vivo.*

As preferred, sequence of derivative of lysyl bradykinin or bradykinin is shown as SEQ ID NO: 14.

The present application also provides compositions containing the above lysyl bradykinin or bradykinin derivatives.

Lysyl bradykinin or bradykinin exerts a series of physiological effects by acting on the corresponding receptors. However, the half-life of lysyl bradykinin and bradykinin *in vivo* is short, only a few seconds, and they are quickly inactivated by kallikrein hydrolysis, which greatly limits druggability. Through polyethylene glycol modification technology, the half-life of lysyl bradykinin or bradykinin *in vivo* is greatly prolonged, and has good biological activity *in vivo* and *in vitro,* such as significantly improving the cerebral infarction symptom in the cerebral ischemia-reperfusion model of MCAO, and solve the problem that lysyl bradykinin or bradykinin alone has very short half-life and cannot be used as drug.

The present invention provides the application of polyethylene glycol modified lysyl bradykinin or bradykinin in the preparation of drug for the treatment, prevention, recovery, and prevention of recurrence of ischemic stroke. The lysyl bradykinin or bradykinin is wild-type lysyl bradykinin or bradykinin, or derivative obtained by modification (not PEGylated modification) of the wild-type lysyl bradykinin or bradykinin. The derivative retains the *in vitro* activity (i.e., binding activity to receptors) of wild-type lysyl bradykinin or bradykinin, includes not only the various mutants mentioned in the examples of the application, but also peptides, fusion proteins (including but not limited to albumin fusion, Fc fusion, etc.) and various forms of modification (except polyethylene glycol modification) on the basis of the Lys-BK/BK mutant described in the application.

Preferably, the variant of lysyl bradykinin or bradykinin and its PEG conjugate is administered by injection or orally.

This application has the following advantages over the prior art:
First, through the polyethylene glycol modification technology, the application greatly extends the half-life of lysyl bradykinin or bradykinin *in vivo,* and at the same time exerts its biological activity well. It solves the problem that lysyl bradykinin or bradykinin has very short half-life and cannot be used as drug. In terms of *in vivo* efficacy, PEG modified lysyl bradykinin or bradykinin or variants can significantly improve the neurological deficit symptom and cerebral infarction size in the cerebral ischemia-reperfusion model of MCAO.

Second, the modified variants of lysyl bradykinin or bradykinin in this application have obvious advantages, showing better stability in serum than the wild-type peptide, indicating prolonged half-life *in vivo,* while maintaining receptor affinity. Moreover, the PEG-modified variants showed better *in vivo* efficacy than PEG-modified wild-type lysyl bradykinin or bradykinin.

Third, the proposed PEG modified lysyl bradykinin or bradykinin show significant efficacy when administered intravenously, subcutaneously, or orally. In particular, subcutaneous injection and oral administration is convenient for clinical use. Patients do not need to go to the hospital regularly for injection, and they can administer drugs at home, which effectively improve the patient compliance and is more suitable for long-term treatment, such as recovery treatment of stroke patients.

### BRIEF DESCRIPTION OF THE DRAWINGS

FIG. 1: UPLC-RP purity analysis of variant Lys-BKm13
FIG. 2: Lys-BK and its variants activated phosphorylation of downstream ERK1/2 and CREB, where β-actin was the internal reference; p- represented the phosphorylation of the corresponding protein; control was buffer; Lys-BK-M06-EH was the mixture of m06 and serum after incubation
FIG. 3: UPLC-RP purity analysis of Lys-BKm13-SPA10K
FIG. 4: Affinity assay of PEG conjugates of Lys-BK and its variant to B2 receptor (reporter gene assay)
FIG. 5: PEG conjugates of Lys-BK and its variant activated phosphorylation of downstream ERK1/2 and CREB, where β-actin was the internal reference; p-represented the phosphorylation of the corresponding protein; control was buffer
FIG. 6: Drug concentration-time curve of Lys-BKm13-SPA10K in SD rats
FIG. 7: Effect of test drug in Example 6 on neurological deficit symptoms and cerebral infarct size.
FIG. 8: Effect of test drug in Example 7 on neurological deficit symptoms and cerebral infarct size.
FIG. 9: Effect of test drug in Example 8 on neurological deficit symptoms and cerebral infarct size.
FIG. 10: Brain section diagram of Example 8
FIG. 11: Effect of test drug in Example 9 on neurological deficit symptoms and cerebral infarct size.
FIG. 12: Brain section diagram of Example 9

### DETAILED DESCRIPTION OF THE INVENTION

Unless otherwise specified, the technical terms or abbreviations of this application have the following meanings:
Lys-BK: wild-type lysyl bradykinin, with sequence identical to native human lysyl bradykinin; The amino acid sequence was KRPPGFSPFR.

BK: wild-type bradykinin, with sequence identical to native human bradykinin; The amino acid sequence was RPPGFSPFR.

Mutant: variant obtained by substitution, insertion, or deletion of some amino acids in wild-type lysyl bradykinin or bradykinin.

Derivatives: including not only the various mutants mentioned in the embodiments of this application, but also peptides, fusion proteins (including but not limited to albumin fusion, Fc fusion, etc.) and various forms of modification (except for PEG modification) on the basis of Lys-BK/BK mutant in this application.

Polyethylene glycol: PEG, usually formed by the polymerization of ethylene oxide, has branched, linear, and multi-arm forms. Ordinary polyethylene glycol has hydroxyl group at each end. If one end is blocked with methyl group, methoxy polyethylene glycol (mPEG) is obtained.

Polyethylene glycol modifier: PEG modifier refers to PEG derivatives with functional groups, which are activated polyethylene glycol and can be used for protein and peptide drug modification. The polyethylene glycol modifier used in this application was purchased from ZonHonBiopharma Institute , Inc or Jenkem Technology Co., LTD. The actual molecular weight of PEG modifier can be 90%-110% of the labeled value. For example, molecular weight of PEG5K can be 4.5-5.5 kDa.
M-SPA-5K: straight-chain monomethoxy polyethylene glycol succinimidyl propionate with molecular weight of about 5kDa, the structure is shown in general formula (1), n is an integer from 98 to 120, 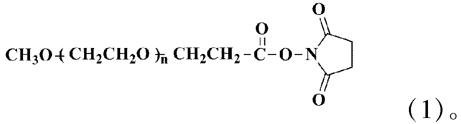
M-SPA-2K: straight-chain monomethoxy polyethylene glycol succinimidyl propionate with molecular weight of about 2kDa, the structure is shown in general formula (1), and n is an integer from 37 to 45.
M-SPA-10K: straight-chain monomethoxy polyethylene glycol succinimidyl propionate with molecular weight of about 10 kDa, the structure is shown in general formula (1), and n is an integer from 200 to 245.
M-SPA-20K: straight-chain monomethoxy polyethylene glycol succinimidyl propionate with molecular weight of about 20kDa and structure as shown in general formula (1), and n is an integer from 405 to 495.
M-SCM-5K: straight-chain monomethoxy polyethylene glycol succinimiyl acetate with molecular weight of about 5kDa, the structure is shown in general formula (2), and n is an integer from 99 to 120. 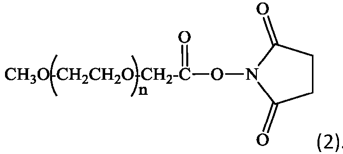
M-SS-5K: straight-chain monomethoxy polyethylene glycol succinimidyl succinate with molecular weight of about 5kDa, the structure is shown in general formula (3), and n is an integer from 98 to 119. 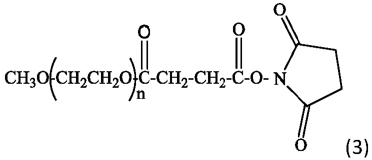
M-YNHS-10K: branched-chain monomethoxy polyethylene glycol succinimidyl acetate with molecular weight of about 10kDa, the structure is shown in general formula (4), n is an integer from 98 to 120,

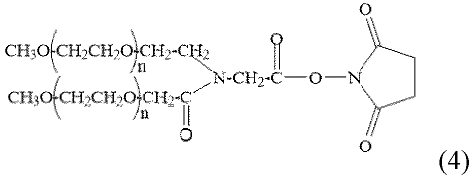

### Example 1 Design, synthesis of lysyl bradykinin/bradykinin variants

Based on the sequence of lysyl bradykinin/bradykinin and the possible degradation pathways *in vivo,* a series of variants were designed (Table 1-1), mainly by replacing natural amino acids with structurally similar natural amino acids and unnatural amino acids (m01~m04 and m07, among which m02 was BK variant). In order to identify the minimum active unit with biological activity/function, partially truncated or extended peptide variants (m05, m06, M08-M11) were designed; Cysteines were introduced (m13, m15, m16) to further improve the antioxidant properties of the variant. To further improve the stability of peptides, lysyl bradykinin/bradykinin monomer was connected in series (m12 was consisted of Lys-BK and BK, m14 was consisted of two Lys-BK). Based on the m13 peptide, m15 and m16 variants were designed to compare the effect of N-terminal extension on drug efficacy.

**Table 1-1 Sequences of lysyl bradykinin/bradykinin variants**

| Peptide | Sequences | SEQ ID NO |
|---|---|---|
| Lys-BK | KRPPGFSPFR | 1 |
| Lys-BKm01 | KRPPG{THI}SPFR | 2 |
| Lys-BKm02 | RPPG{THI}SPFR | 3 |
| Lys-BKm03 | KRPPGHSPFR | 4 |
| Lys-BKm04 | KRPPGFSPHR | 5 |
| Lys-BKm05 | KRPPG{THI}SPF | 6 |
| Lys-BKm06 | KRPPG{THI}SPFRR | 7 |
| Lys-BKm07 | KRPPG{THI}SP{THI}R | 8 |
| Lys-BKm08 | PG{THI}SPFR | 9 |
| Lys-BKm09 | {THI}SPFR | 10 |
| Lys-BKm10 | SPFR | 11 |
| Lys-BKm11 | KRRPPG{THI}SPFR | 12 |
| Lys-BKm12 | KRPPG{THI}SPFRRPPG{THI}SPFR | 13 |
| Lys-BKm13 | CRRPPG{THI}SPFR | 14 |
| Lys-BKm14 | KRPPG{THI}SPFRKRPPG{THI}SPFR | 15 |
| Lys-BKm15 | CRPPG{THI}SPFR | 16 |
| Lys-BKm16 | CRRRPPG{THI}SPFR | 17 |

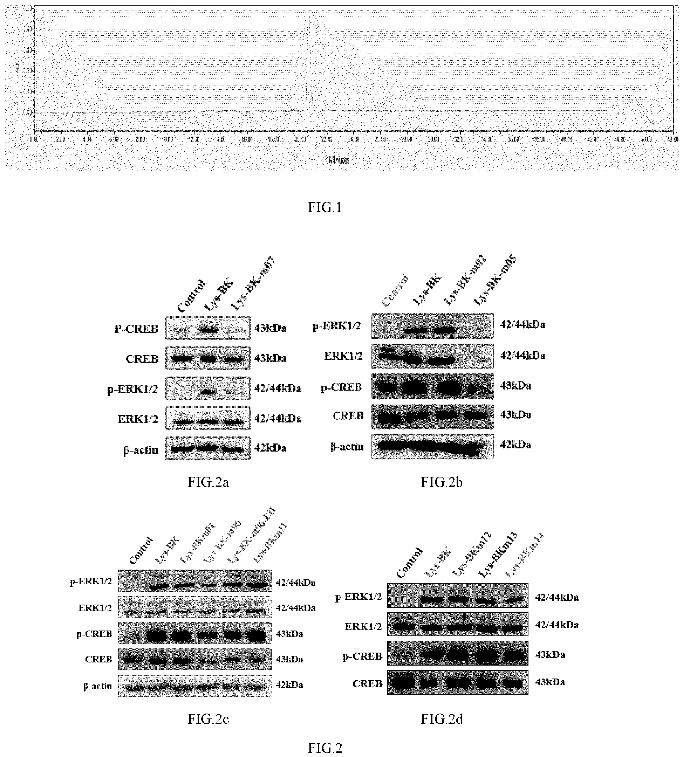

According to general rule 0512 of "Chinese Pharmacopoeia" 2020 edition, high performance liquid chromatography was used for detection. The chromatographic column was C18-RP (reverse phase chromatography), and the mobile phase was A: 0.1% TFA/H₂O. B: 0.1% TFA/ACN, column ACQUITY BEH C18: (1.7µm,2.1×50mm), acquisition condition 214nm. The purity of all peptides was > 95%. In the case of Lys-BKm13, the purity analysis spectrum is shown in figure. 1.

### Example 2 Stability studies of lysyl bradykinin/bradykinin and its variants

Assess the stability of lysyl bradykinin/bradykinin by evaluating its stability in serum system *in vitro.* 2mg/ml peptide and SD rat serum were mixed at volume ratio of 85: 15, incubated at 37°C for 30min, 60min, 90min, 120min, 180min, etc, terminate by adding 3% trichloroacetic acid (50% volume of the above mixture system with peptide and serum) and effectively remove matrix interference in the serum. Further monitor the degradation of peptide samples in the serum system to evaluate its stability basing on UPLC-RP-UV₂₁₄ₙₘ method. RP analysis method: Protein BEH C4 2.1 × 150mm as chromatographic column, 1.7 µm, column temperature 30 °C, mobile phase A: 0.1% TFA/H₂O, mobile phase B: 0.1% TFA/ACN, gradient: 0-5min, 5%B; 5-10min, 5-40% B; 10-30min, 40-60%B; 30-32min, 60-100% B; 32 to 35min, 5% B.

**Table 2-1 Stability of lysyl bradykinin and its variants in serum system**

| Peptide | Residual time in serum system |
|---|---|
| Lys-BK | 30~60min |
| Lys-BKm01 | 60~90min |
| Lys-BKm02 | 60-90min |
| Lys-BKm03 | 60~90min |
| Lys-BKm04 | >180min |
| Lys-BKm05 | >180min |
| Lys-BKm06 | 60~90min |
| Lys-BKm07 | 60~90min |
| Lys-BKm11 | 60~90min |
| Lys-BKm12 | 90-120min |
| Lys-BKm13 | 60~90min |
| Lys-BKm14 | 90~120min |
| Lys-BKm15 | 60~90min |
| Lys-BKm16 | 60~90min |

The results in Table 2-1 showed that the designed polypeptide variants had better stability in serum than the wild type lysyl bradykinin, which also indicated that they had certain advantages in stability and drug efficacy *in vivo.*

### Example 3 Evaluation of in vitro activity of lysyl bradykinin/bradykinin and its variants

### I. Affinity activity to B2 receptor based on reporter gene assay

The pharmacological action of lysyl bradykinin/bradykinin *in vivo* is achieved by binding to B2 receptors and activating downstream signaling pathways

The cell line B2-NFAT-CHO-K1 stably transfected with B2 receptor reporter gene was constructed based on CHO-K1 host cells and the cell library was constructed. The B2-NFAT-CHO-K1 cell library was used to evaluate the binding activity of lysyl bradykinin/bradykinin or its variants or PEG modified products to B2 receptor *in vitro.*

Detection methods: The cells were cultured in 96-well plates at 5 × 10⁴ cells/well, 37°C, 5% CO₂ for 16-20 hours, and then gradient diluted standard and test solutions were added. After 5 hours, added Bio-Lite luciferase detection reagent and the chemiluminescence intensity was measured. GraphPad was used to make four-parameter equation curve and calculate the relative activity according to the EC50 value. Table 3-1 listed the relative affinity activity of the peptide variants to B2 receptor, among which Lys-BKm11, Lys-BKm13, Lys-BKm15 and Lys-BKm16 had higher activity than the wild type Lys-BK.

**Table 3-1 Relative affinity activity of Lys-BK and its variants to B2 receptor**

| Peptide | Relative affinity activity | Peptide | Relative affinity activity |
|---|---|---|---|
| Lys-BK | 100% | Lys-BKm08 | <10% |
| Lys-BKm01 | 100% | Lys-BKm09 | 0% |
| Lys-BKm02 | 100% | Lys-BKm10 | 0% |
| Lys-BKm03 | <10% | Lys-BKm11 | 110% |
| Lys-BKm04 | <10% | Lys-BKm12 | 100% |
| Lys-BKm05 | <10% | Lys-BKm13 | 115% |
| Lys-BKm06 | 90% | Lys-BKm14 | 100% |
| Lys-BKm07 | <10% | Lys-BKm15 | 125% |
| | | Lys-BKm16 | 120% |

### II. Activity analysis based on key target proteins in downstream signaling pathways

B2-NFAT-CHO-K1,which was CHO-K1 cells overexpressing B2 receptor, was *in vitro* intervened with Lys-BK/BK or its different variants. And the changes of p-CREB and p-ERK1/2, which was B2 receptor downstream signals, were detected by WB method to compare the activity difference between the test substances. Extracellular regulated protein kinases 1/2 (ERK1/2), cAMP-response element binding protein (CRBE) are direct signal molecules after B2R activation. After stroke, the activation of CREB and ERK1/2 can prevent the inflammatory response and neuronal damage after ischemia, so the activation level of CREB and ERK1/2 can reflect B2R activating ability of the test substance. At the same time, the significance of the neuroprotective effect is reflected.

CHO-K1(B2R+) cells in logarithmic growth phase were collected and adjusted to 3×10⁶ cells/mL with medium and seeded in cell culture plates at 3×10⁵ cells/ well. The cells were cultured in a CO₂ incubator at 37.0°C and 5.0% CO₂ for 24-48 hours. When the cell density was more than 90%, the cells were washed with PBS, and 1ml 1µM Lys-BK/BK or its variant sample was added to each well, while the control group was F12 medium. The cell culture plates were incubated at 37.0°C, 5.0% CO₂ for 5min, and the cell culture plates were washed with PBS. Protein lysate (RIPA, PMSF, phosphatase inhibitor) was added, 80µL/ well, and the bottom of the well was ground back and back with a scraping rod to accelerate cell lysis. The cell debris and lysate was transferred to a centrifuge tube and centrifuged at 12000rpm, 4°C for 10min. Draw the supernatant, mix with 5×Loading Buffer at volume ratio of 4:1, and boil in metal bath at 100°C for 10min. The same amount of protein samples were loaded into 10% SDS-PAGE for gel running, membrane transfer, blocking, antibody incubation, and luminescence identification.

As shown in FIG. 2, Lys-BK/BK can activate the phosphorylation of downstream ERK1/2 and CREB proteins, which was consistent with the action mechanism of this drug. The *in vitro* biological activity of the samples was qualitatively evaluated by judging the phosphorylation level of the downstream ERK1/2 and CREB proteins. The evaluation results showed that Lys-BKm05 and Lys-BKm07 lost the activity of activating downstream pathways, while LBKm01, m02, m06, m11, m12, m13, m14, m15 and m16 all showed the activity of activating downstream pathways.

The above *in vitro* activity evaluation results showed that lysyl bradykinin/bradykinin with C-terminal truncation lost B2R affinity and lost activity of activating downstream pathway. Lysyl bradykinin missing N-terminal lysine did not affect B2R affinity and activity of activating downstream pathway. Lysyl bradykinin missing other N-terminal amino acids lost B2R affinity and activity of activating downstream pathway. The minimum active unit was RPPGFSPFR. Both N-terminal lengthening (m11) or introduction of cysteine (m13) can increase B2R affinity activity. Figure 2d shows that the phosphorylation of CREB protein in m12, 13, and 14 groups was significantly higher than that in LBK group, revealing that m12, 13, and 14 could better activate the downstream pathways and show better *in vitro* activity than LBK.

### Example 4 Preparation of PEG conjugates of lysyl bradykinin/bradykinin and its variants

PEG modification of lysyl bradykinin/bradykinin and its variants was carried out by conventional methods in the art, specific to particular variants and PEG modifiers, with slight changes in some parameters, which can be obtained by those skilled in the art through a limited number of experiments, and there are no technical obstacles. Taking M-SPA-5K modified wild-type BK as an example, the preparation process was as follows:

### I. Preparation of PEG conjugates

Peptide sample was dissolved with pH7.0 sodium dihydrogen phosphate/disodium hydrogen phosphate buffer so that the final concentration was 10mg/mL. PEG was added to the peptide solution, and the mass ratio of peptide to M-SPA-5K was 1:7, and the mixture was stirred slowly until it was evenly mixed. The reaction was carried out at 2-8°C for 1h.

### II. Purification of the reaction mixture

In the first purification step, the chromatographic conditions were as follows: GE Superdex75 medium was used as the purification filler, and the purification mobile phase was pH7.0-7.5, 20mM sodium dihydrogen phosphate/disodium hydrogen phosphate buffer containing 0.2M NaCl.

Sample loading: After the reaction, the above reaction mixture was directly loaded.

Elution: after sample loading, the chromatographic column was washed with mobile phase of no less than 1.5 column volume, and eluted samples were collected step by step according to the trend of UV₂₁₄ₙₘ.

In the second purification step, the chromatographic conditions were as follows:
C18 reversed-phase column achieved separation based on the polarity difference, the product purification yield can reach more than 80%, and the free PEG can be completely removed.

### III. Purity analysis of PEG conjugate

### (1)HPLC purity analysis

The detection was performed by high performance liquid chromatography (HPLC) according to the General rule 0512 of Chinese Pharmacopoeia (2020 Edition). The chromatographic type was SEC (size-exclusion chromatogrphy chromatography), the mobile phase was 20mM PB7.0+10% IPA, the chromatographic column was TSK G2000, and the collection condition was 214nm.

RP purity analysis conditions were as follows: chromatographic column was Protein BEH C4 2.1 × 150mm, 1.7µm, mobile phase A was 0.1% TFA/H₂O, and mobile phase B was 0.1% TFA/ACN.

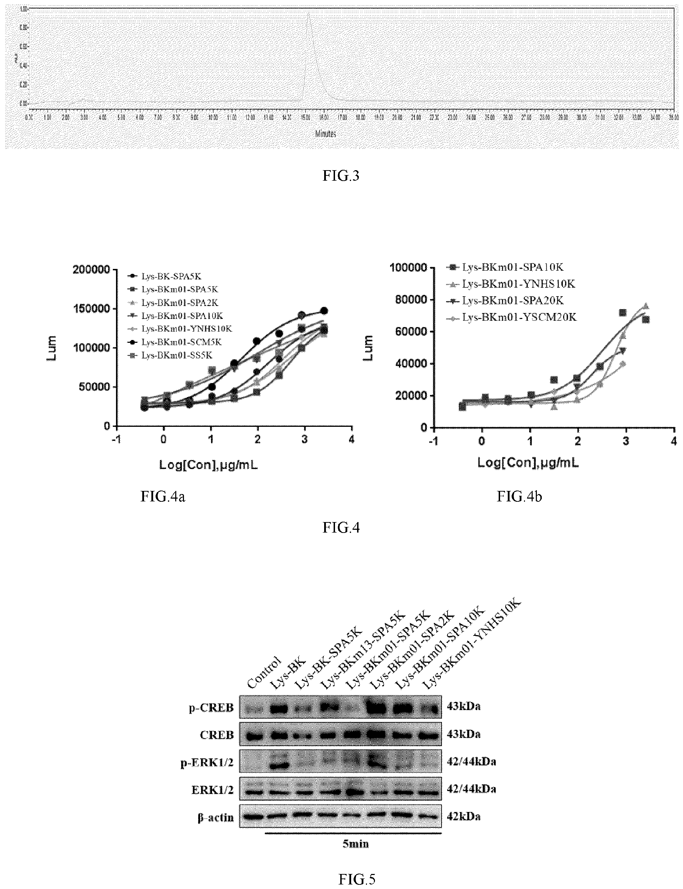

The purity results showed that the PEG modified lysyl bradykinin variants had uniform peaks, no obvious impurity peaks, and the purity was > 95%. In the case of Lys-BKm13-SPA10K, the purity analysis spectrum was shown in Figure 3.

### (2) Purity analysis by SDS-PAGE

The sample purity was determined by SDS-polyacrylamide gel electrophoresis method, the fifth method of electrophoresis method in Chinese Pharmacopoeia 0541 version 2020, and 12.5% SDS-PAGE was used for sample detection.

The results of electrophoresis showed that the prepared PEG modified lysyl bradykinin variants had uniform bands, no heterozygous bands were observed, and the purity was > 95%.

In addition, lysyl bradykinin/bradykinin or its variants only had a theoretically modification site at the N-terminus, so the PEG modified products were all N-terminal single-modified.

### Example 5 In vitro evaluation of PEG conjugates of lysyl bradykinin/bradykinin and its variants

### I. Stability

The stability of PEG conjugates of lysyl bradykinin and its variants was indirectly evaluated by evaluating their stability in serum system *in vitro,* as determined in Example 2. PEG conjugates of lysyl bradykinin and its variants had residual time of more than 6h in the serum system *in vitro* (Table 5-1), indicating significant increase in stability. The stability of the branched chain conjugate Lys-BKm01-YNHS10K was better than that of the straight chain conjugate Lys-BKm01-SPA10K with the same molecular weight of PEG.

**Table 5-1 Stability of PEG conjugates of lysyl bradykinin and its variants in the serum system**

| Peptides | Residual time in serum system |
|---|---|
| Lys-BK-SPA5K | >6h |
| Lys-BKm01-SPA2K | >6h |
| Lys-BKm01-SPA5K | >6h |
| Lys-BKm01-SPA10K | >6h |
| Lys-BKm01-YNHS10K | >12h |
| Lys-BKm01-SCM5K | >6h |
| Lys-BKm 01-SS5K | >6h |

### II. In vitro activity

*In vitro* B2-receptor affinity activity and activity of activating downstream signaling pathway of PEG conjugates of lysyl bradykinin and its variants were tested, as in Example 3. Lysyl bradykinin or its variants after PEG modification still had affinity to B2 receptor (Figure 4a, 4b), and still can activate the phosphorylation of key proteins, which were downstream signaling pathways of B2 receptors (Figure 5). Conjugates using PEG modifiers with different structural types and molecular weights, including 2K to 20K, straight chain and branch, and different structures (SPA, SCM, SS, etc.) still retained the binding activity to B2 receptor. The results showed that the activity of straight-chain PEG conjugates was higher than that of branched-chain PEG conjugates with the same molecular weight, and this result was true for both 10K and 20K PEG. As shown in Figure 4a, the EC₅₀ value of Lys-BKm01-SPA10K and Lys-BKm01-YNHS10K were 90.8µg/ml and 346.6µg/ml, respectively, indicating that the linear PEG conjugate was nearly 4-fold more active than the branched PEG conjugate. Among 2K, 5K, 10K and 20K PEG conjugates with the same PEG structure type, the activity of 2K and 10K PEG conjugates was relatively higher.

The activity results of activating the downstream signaling pathway of PEG conjugates of lysyl bradykinin and its variants (FIG. 5) showed that PEG conjugates of the peptides retained the activity of activating the phosphorylation of ERK1/2 and CREB, which was downstream of the B2 receptor, although the activity decreased compared with Lys-BK, especially ERK1/2 phosphorylation. With the same SPA5K-PEG modifier, Lys-BKm13-SPA5K had the highest activity among different peptide variants. With the same peptide variant Lys-BKm01, 2K and 10K PEG conjugates showed the highest activity, and the linear PEG conjugate Lys-BKM01-SPA10K showed higher activity than the branched PEG conjugate Lys-BKM01-YNHS10K.

### Example 6 In vivo metabolism studies of PEG conjugates of lysyl bradykinin

### I. Experimental methods

SD rats were subcutaneously injected with 0.5mg/kg lysyl bradykinin PEG conjugate (Lys-BKm13-SPA10K as an example). Plasma samples were collected before administration and 5min, 15min, 0.5h, 1h, 2h, 4h, 8h, 24h, 48h and 72h after administration. The concentration of Lys-BKm13-SPA10K in plasma samples was measured by specific ELISA in ng/ml.

### II. Detection methods

1. Coating: streptavidin (4ug/ml) was coated into the ELISA plate, 2-8°C overnight; add blocking solution after washing once, block at 37°C for 2 hours, and set aside.
2. Capture: commercial anti-PEG-biotin was diluted to 0.5ug/ml with blocking solution and added to the above ELISA plate, 37°C 300rpm for 2 hours. Wash three times with wash solution and pat dry.
3. Sample addition: dilute the standard material or sample to the corresponding concentration; then dilute 10 times with blocking solution and added to the ELISA plate. 37°C 300rpm for 1.5 hours. Wash three times with wash solution and pat dry.
4. The detection material: HRP labeled anti-Lys-BKM13-SPA10K specific antibody was diluted 1000 times and added to the above ELISA plate, 37°C 300rpm for 1.5 hours. Wash three times with wash solution and pat dry.
5. Color development: TMB was added to the above ELISA plate, color development at 37°C for 15min, and 2M H₂SO₄ was added.
6. Reading plate: OD450 and OD630 were read in the ELISA plate reader.

Drug concentrations in samples were calculated by four-parameter fitting using OriginPro 9. 1.

### III. Test results

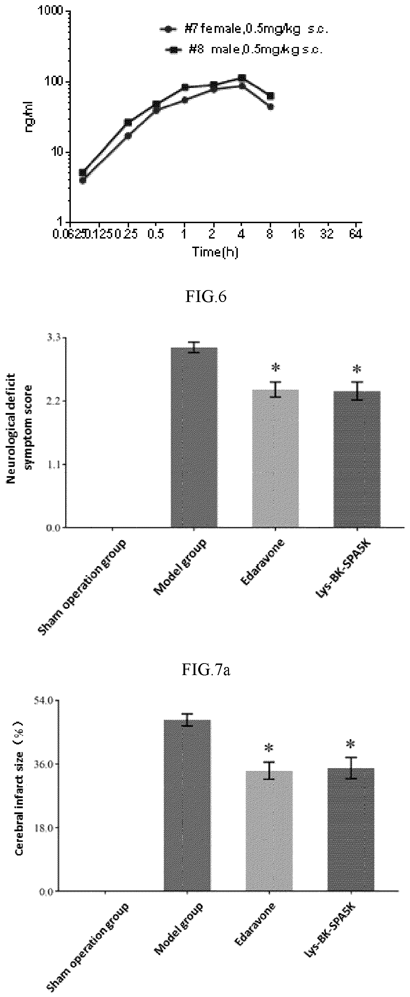

The drug concentration-time curve of Lys-BKm13-SPA10K in SD rats was shown in FIG. 6. After single subcutaneous administration (0.5mg/kg), the Cmax of male and female rats reached 88.07ng/ml and 114.68ng/ml respectively, 4 hours after administration. After subcutaneous administration of 30mg of icatibant (structural analogue of bradykinin) in adults (reference: Efficacy,pharmacokinetics,and safety of icatibant for the treatment of Japanese patients with an acute attack of hereditary angioedema:A phase 3 open-label study), the peak blood concentration reached 405ng/ml 1.79 hours later. With the same dose (equivalent dose according to body surface area), Lys-BKm13-SPA10K had higher Cmax and longer time to peak, which indicated that Lys-BKM13-SPA10K had higher bioavailability.

### Example 7 In vivo activity evaluation of PEG conjugates of lysyl bradykinin (unmutated)

### 1. Model preparation

SD rats, male, SPF grade, weighing 270-300g. The middle cerebral artery occlusion (MCAO) of cerebral ischemia-reperfusion model was prepared by using the intraluminal suture method in rats. Animals were anesthetized with gas (isoflurane), and then fixed them in a supine position. The skin was disinfected, and the right common carotid artery, external carotid artery, and internal carotid artery were separated from the midline incision in the neck. The vagus nerve was gently peeled off, and the external carotid artery was ligated and cut. The common carotid artery was clamped near the proximal end, and an incision was made from the distal end of the ligature line of the external carotid artery. The suture(model 2438-A5, purchased from Beijing Xinong Technology Co., LTD.) was inserted and passed through the bifurcation of the common carotid artery into the internal carotid artery. The suture was then slowly inserted until slight resistance was encountered (about 20mm from the bifurcation), blocking the blood supply to the middle cerebral artery. The neck skin was sutured, disinfected, and the rat was returned to the cage. After 90 minutes of ischemia, the rat was anesthetized again, fixed on the rat board, and the neck skin was cut open to find the suture, which was gently removed. Reperfusion was performed, and the neck skin was sutured, disinfected, and the rat was returned to the cage for feeding.

Grouping and administration:
The rats were divided into sham operation group, model group (administered PBS intravenously), Edaravone group (administered intravenously, 6mg/kg), and PEG conjugate of lysyl bradykinin group (administered intravenously, 3mg/kg). The rats were injected once after 2 hours of reperfusion.

### 2. Evaluation of neurological deficits

The modified Bederson method was used to evaluate neurological defect symptoms.

| |
|---|
| 0: When the tail is suspended, both forelimbs of the animal extend towards the floor without other behavioral deficits. |
| 1: When the tail is suspended, the forelimb of the animal contralateral to the surgery (left) shows wrist and elbow flexion, shoulder internal rotation, elbow abduction, and tight adhesion to the chest wall. |
| 2: When the animal is placed on a smooth plate, the resistance is reduced when the operated side shoulder is moved to the contralateral side. |
| 3: When the animal walks freely, it circles or turns to the opposite side of the surgery. |
| 4: Limb paralysis, no spontaneous movement. |

### 3. Measurement of Cerebral Infarction Area

Animals were anesthetized with 10% chloral hydrate, the brain tissue was removed, the olfactory bulbs, cerebellum and lower brainstem were removed, and the bloodstain on brain surface was washed with SPSS. After removing surface residual water, the brain tissue was placed at -80°C for 7 minutes, took out and cut into coronal sections perpendicular to the visual cross plane, and sliced at intervals of 2mm to the posterior direction. The brain slices were placed in fresh TTC (20g/L) dye solution prepared with SPSS at 37°C for 90 minutes. Normal brain tissue was stained deep red, while ischemic brain tissue appeared pale. After washing with SPSS, the brain slices were quickly arranged in sequence from front to back, residual water on the surface was absorbed, and photographed. The image analysis software (Image Tool) was used to delineate the ischemic area (white area) and the right side area on the photos for statistical analysis. The percentage of cerebral infarction area was calculated using the following formula: Cerebral infarction area (%) = 100 × total ischemic area/total right hemisphere area.

### 4. Results

### 1). The effect of the test substance on the neurological defects symptoms

As shown in Figure 7a, the positive drug group (Edaravone) showed significant improvement in neurological deficit symptoms compared with the model group. Compared with the model group, the PEG conjugate of lysyl bradykinin Lys-BK-SPA5K group had significant improvement in neurological deficit symptoms, and the difference was statistically significant.

**Table 6-1 Effects of Lys-BK-SPA5K et al on neurological deficit symptoms**

| Groups | Total animal | Death | Discard | Sample | Neurological deficit symptom score |
|---|---|---|---|---|---|
| Sham operation group | 8 | 0 | 0 | 8 | 0.00 ± 0.00 |
| Model group | 20 | 5 | 0 | 15 | 3.13 ± 0.09 |
| Edaravone group | 20 | 5 | 0 | 15 | 2.40 ± 0.13* |
| Lys-BK-SPASK group | 20 | 4 | 0 | 16 | 2.38 ± 0.15* |

| | | | | | |
|---|---|---|---|---|---|
| Mean ± SE. *P<0.05, compared with model group. | | | | | |

### 2). The effect of the test substance on the cerebral infarction area

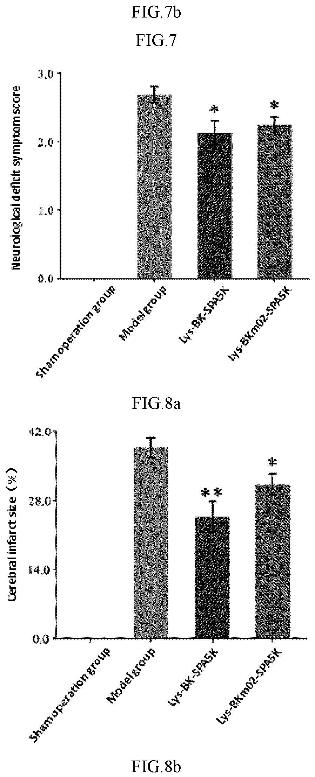

As shown in Figure 7b, the positive drug group (Edaravone) had significant improvement in cerebral infarct size compared with the model group. Compared with the model group, the PEG conjugate of lysyl bradykinin group had significant improvement in cerebral infarction area, and the difference was statistically significant.

**Table 6-2 Effect of Lys-BK-SPA5K et al on cerebral infarct size (%)**

| Groups | Total animal | Death | Discard | Sample | Cerebral infarct size (%) |
|---|---|---|---|---|---|
| Sham operation group | 8 | 0 | 0 | 8 | 0.00 ± 0.00 |
| Model group | 20 | 5 | 0 | 15 | 48.50 ± 1.66 |
| Edaravone group | 20 | 5 | 0 | 15 | 34.12 ± 2.35* |
| Lys-BK-SPASK group | 20 | 4 | 0 | 16 | 34.84 ± 2.90* |

| | | | | | |
|---|---|---|---|---|---|
| Mean ± SE. *P<0.05, compared with model group. | | | | | |

### Example 8 In vivo activity evaluation of PEG conjugate of lysyl bradykinin/bradykinin variant

PEG conjugates of bradykinin lysyl and its variants were evaluated *in vivo* in MCAO models.

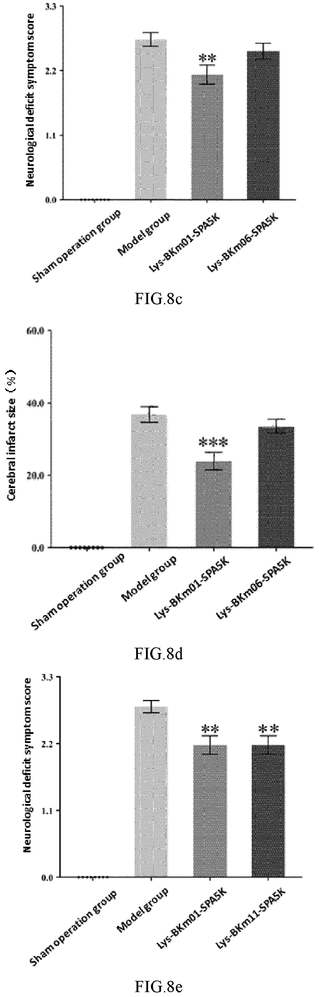

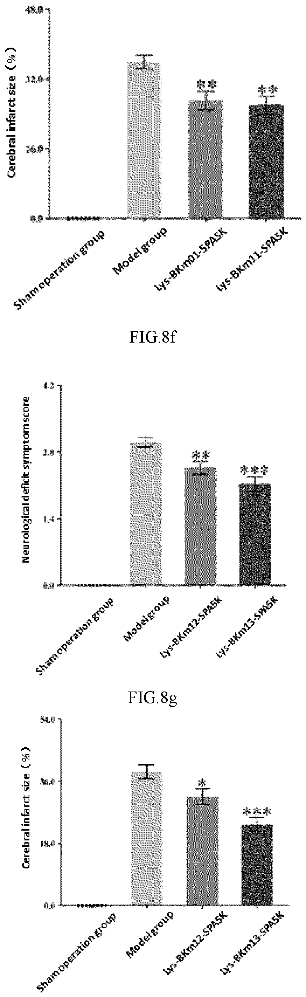

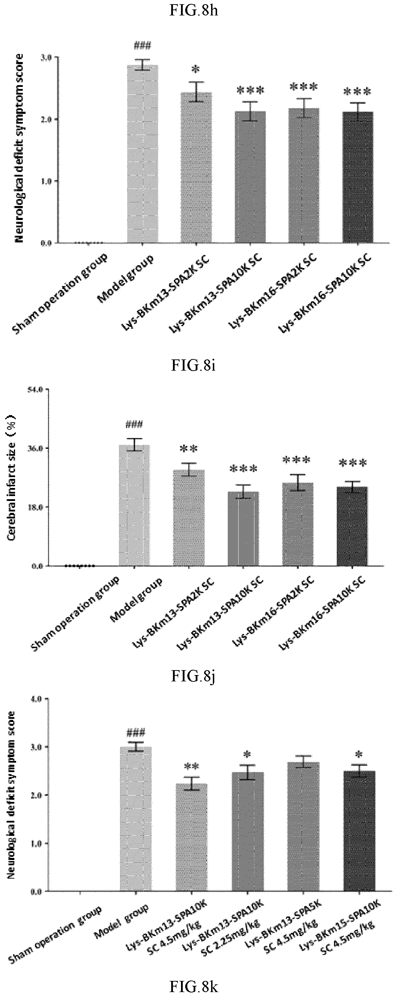

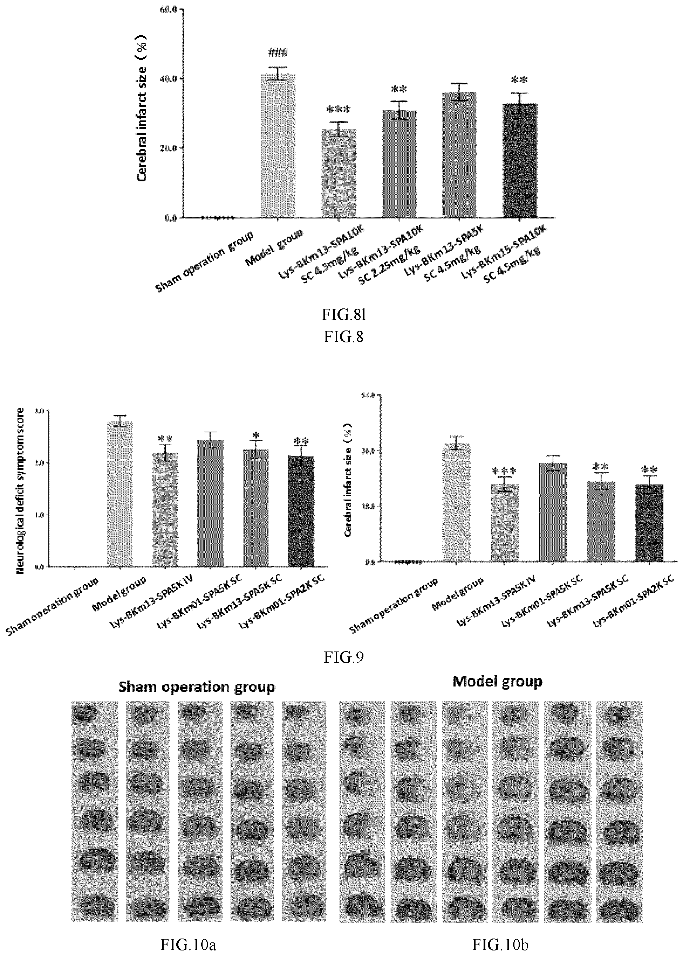

Except for Table 7-6, the dosage of the other groups was 3mg/kg (all the dosage was calculated based on peptides). The administration methods included intravenous injection and subcutaneous injection, and the injection was given once after 2 hours of reperfusion. Other specific experimental schemes refered to Example 7, and the results were shown in Figure 8 and Table 7.

**Table 7-1. Effect of Test Subjects on Neurological Deficit Symptoms and Cerebral Infarct Size (%)**

| Groups | Total animal | Death | Discard | Sample | Neurological deficit symptom score | Cerebral infarct size (%) |
|---|---|---|---|---|---|---|
| Sham operation group | 8 | 0 | 0 | 8 | 0.00 ± 0.00 | 0.00 ± 0.00 |
| Model group | 18 | 2 | 0 | 16 | 2.69 ± 0.12 | 38.73 ± 1.99 |
| Lys-BK-SPASK group (Intravenous injection) | 18 | 2 | 0 | 16 | 2.13 ± 0.18* | 24.73±3.15** |
| Lys-BKm02-SPASK group (Intravenous injection) | 18 | 2 | 0 | 16 | 2.25 ± 0.11* | 31.36 ± 2.13* |

| | | | | | | |
|---|---|---|---|---|---|---|
| Mean ± SE. *P<0.05, **P<0.01, ***P<0.001 compared with the model group. | | | | | | |

**Table 7-2. Effect of Test Subjects on Neurological Deficit Symptoms and Cerebral Infarct Size (%)**

| Groups | Total animal | Death | Discard | Sample | Neurological deficit symptom score | Cerebral infarct size (%) |
|---|---|---|---|---|---|---|
| Sham operation group | 8 | 0 | 0 | 8 | 0.00 ± 0.00 | 0.00 ± 0.00 |
| Model group | 20 | 5 | 0 | 15 | 2.73 ± 0.12 | 36.77± 2.17 |
| Lys-BKm01-SPA5K group (Intravenous injection) | 20 | 5 | 0 | 15 | 2.13 ± 0.17** | 23.91±2.38*** |
| Lys-BKm06-SPASK group (Intravenous injection) | 20 | 5 | 0 | 15 | 2.53 ± 0.13 | 33.44 ± 1.95 |

| | | | | | | |
|---|---|---|---|---|---|---|
| Mean ± SE. *P<0.05, **P<0.01, ***P<0.001 compared with the model group. | | | | | | |

**Table 7-3. Effect of Test Subjects on Neurological Deficit Symptoms and Cerebral Infarct Size (%)**

| Groups | Total animal | Death | Discard | Sample | Neurological deficit symptom score | Cerebral infarct size (%) |
|---|---|---|---|---|---|---|
| Sham operation group | 8 | 0 | 0 | 8 | 0.00 ± 0.00 | 0.00 ± 0.00 |
| Model group | 20 | 4 | 0 | 16 | 2.81 ± 0.10 | 35.95 ± 1.51 |
| Lys-BKm01-SPA5K group (Intravenous injection) | 20 | 3 | 0 | 17 | 2.18 ± 0.18** | 27.04 ± 2.06** |
| Lys-BKm11-SPA5K group (Intravenous injection) | 20 | 3 | 0 | 17 | 2.18 ± 0.15** | 25.89 ± 2.12** |

| | | | | | | |
|---|---|---|---|---|---|---|
| Mean ± SE. *P<0.05, **P<0.01, ***P<0.001 compared with the model group. | | | | | | |

**Table 7-4. Effect of Test Subjects on Neurological Deficit Symptoms and Cerebral Infarct size (%)**

| Groups | Total animal | Death | Discard | Sample | Neurological deficit symptom score | Cerebral infarct size (%) |
|---|---|---|---|---|---|---|
| Sham operation group | 8 | 0 | 0 | 8 | 0.00 ± 0.00 | 0.00 ± 0.00 |
| Model group | 20 | 5 | 0 | 15 | 3.00 ± 0.10 | 38.79 ± 2.01 |
| Lys-BKm12-SPASK group (Intravenous injection) | 20 | 5 | 0 | 15 | 2.47 ± 0.13** | 31.61 ± 2.24* |
| Lys-BKm13-SPASK group (Intravenous injection) | 20 | 4 | 0 | 16 | 2.13 ± 0.15*** | 23.54 ± 1.99*** |

| | | | | | | |
|---|---|---|---|---|---|---|
| Mean ± SE. *P<0.05, **P<0.01, ***P<0.001 compared with the model group. | | | | | | |

**Table 7-5. Effect of Test Subjects on Neurological Deficit Symptoms and Cerebral Infarct Size (%)**

| Groups | Total animal | Death | Discard | Sample | Neurological deficit symptom score | Cerebral infarct size (%) |
|---|---|---|---|---|---|---|
| Sham operation group | 8 | 0 | 0 | 8 | 0.00 ± 0.00 | 0.00 ± 0.00 |
| Model group | 20 | 4 | 0 | 16 | 2.88 ± 0.09 | 37.02 ± 1.88 |
| Lys-BKm13-SPA2K group (Subcutaneous injection) | 20 | 4 | 0 | 16 | 2.44 ± 0.16* | 29.43 ± 1.98** |
| Lys-BKm13-SPA10K group (Subcutaneous injection) | 20 | 4 | 0 | 16 | 2.13 ± 0.15*** | 22.70 ± 2.05*** |
| Lys-BKm16-SPA2K group (Subcutaneous injection) | 20 | 3 | 0 | 17 | 2.18 ± 0.15*** | 25.48 ± 2.42*** |
| Lys-BKm16-SPA10K group (Subcutaneous injection) | 20 | 3 | 0 | 17 | 2.12 ± 0.15*** | 24.14 ± 1.70*** |

| | | | | | | |
|---|---|---|---|---|---|---|
| Mean ± SE. *P<0.05, **P<0.01, ***P<0.001 compared with the model group. | | | | | | |

**Table 7-6. Effect of Test Subjects on Neurological Deficit Symptoms and Cerebral Infarct Size (%)**

| Groups | Total animal | Death | Discard | Sample | Neurological deficit symptom score | Cerebral infarct size (%) |
|---|---|---|---|---|---|---|
| Sham operation group | 8 | 0 | 0 | 8 | 0.00 ± 0.00 | 0.00 ± 0.00 |
| Model group | 20 | 4 | 0 | 16 | 3.00 ± 0.09 | 41.40 ± 1.83 |
| Lys-BKm13-SPA10K group (Subcutaneous injection 4.5mg/kg) | 20 | 3 | 0 | 17 | 2.24 ± 0.14** | 25.32 ± 2.11*** |
| Lys-BKm13-SPA10K group (Subcutaneous injection 2.25mg/kg) | 20 | 3 | 0 | 17 | 2.47 ± 0.15* | 30.81 ± 2.59** |
| Lys-BKm13-SPASK group (Subcutaneous injection 4.5mg/kg) | 20 | 4 | 0 | 16 | 2.69 ± 0.12 | 36.07 ± 2.47 |
| Lys-BKm15-SPA10K group (Subcutaneous injection 4.5mg/kg) | 20 | 4 | 0 | 16 | 2.50 ± 0.13* | 32.76 ± 2.93** |

| | | | | | | |
|---|---|---|---|---|---|---|
| Mean ± SE. *P<0.05, **P<0.01, ***P<0.001 compared with the model group. | | | | | | |

### Example 9 Evaluation of in vivo activity of different administration methods and conjugates with different PEG modifiers

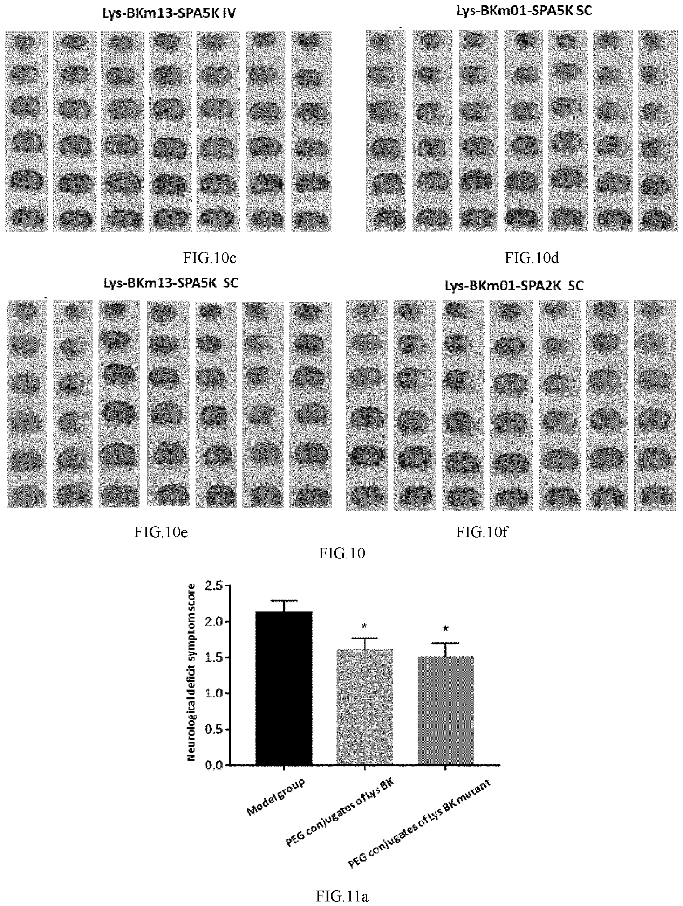

Based on the same model, the effect of PEG conjugate of lysyl bradykinin variant was investigated by both intravenous and subcutaneous injection at dose of 4.5mg/kg (the dose was calculated based on polypeptide). Other specific experimental schemes were referred to Example 7, and the results were shown in Figs. 9, 10, and Table 8-1. The Lys-BKm13-SPA5K group had significant improvement in neurological deficit symptoms and cerebral infarction area compared with the model group by intravenous injection and subcutaneous injection, and the difference was statistically significant. Lys-BKm01-SPA2K was more effective than Lys-BKm01-SPASK *in vivo.*

**Table 8-1 Effect of test subjects on neurological deficit symptoms and cerebral infarct size (%)**

| Groups | Total animal | Death | Discard | Sample | Neurological deficit symptom score | Cerebral infarct size (%) |
|---|---|---|---|---|---|---|
| Sham operation group | 8 | 0 | 0 | 8 | 0.00 ± 0.00 | 0.00 ± 0.00 |
| Model group | 20 | 5 | 0 | 15 | 2.80 ± 0.11 | 38.45 ± 2.16 |
| Lys-BKm13-SPA5K Intravenous injection | 20 | 4 | 0 | 16 | 2.19 ± 0.16** | 25.16± 2.36*** |
| Lys-B Km 01-S PA5 K subcutaneous injection | 20 | 4 | 0 | 16 | 2.44± 0.16* | 31.95 ± 2.40 |
| Lys-BKm13-SPA5K subcutaneous injection | 20 | 4 | 0 | 16 | 2.25 ± 0.17* | 26.06 ± 2.69** |
| Lys-BKm01-SPA2K subcutaneous injection | 20 | 5 | 0 | 15 | 2.13 ± 0.19** | 24.92 ± 2.89** |

| | | | | | | |
|---|---|---|---|---|---|---|
| Mean ± SE. *P<0.05, **P<0.01, ***P<0.001 compared with the model group. | | | | | | |

### Example 10 In vivo activity evaluation of oral administration of PEG conjugates of lysyl bradykinin/bradykinin variant

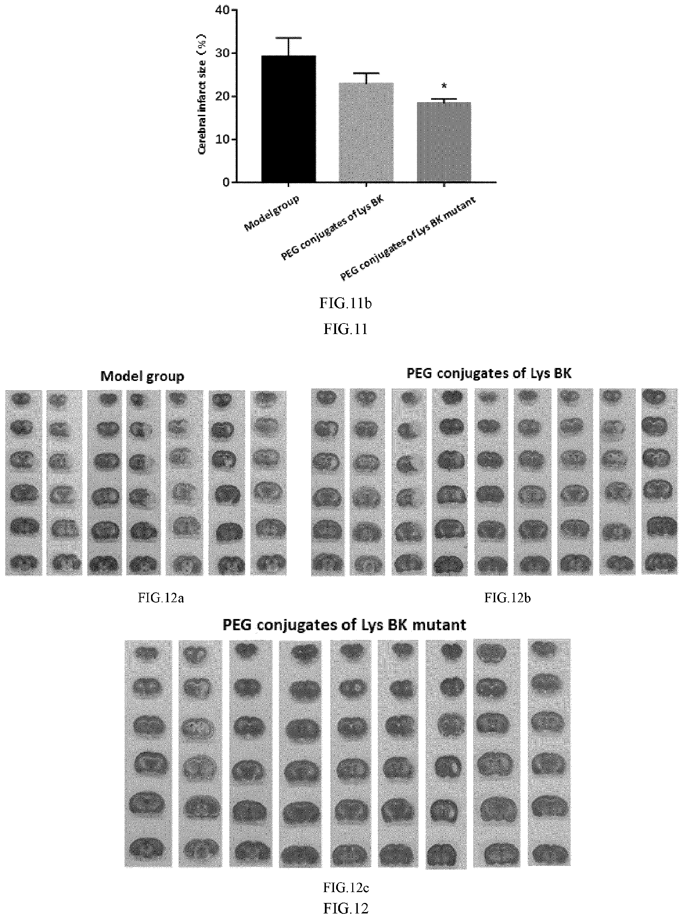

The efficacy of PEG conjugates of lysyl bradykinin and its variants in MCAO model after oral administration was compared. In this example, lysyl bradykinin was wild-type unmutated, variant was M01, PEG was M-SPA-5K, administered once at dose of 6mg/kg (based on polypeptide). The results were shown in Figs. 11, 12, and Table 9-1.

### 1, The effect of the test substance on the neurological defects symptoms

As shown in Table 9-1, PEG conjugates of lysyl bradykinin mutants showed significant improvement in neurological deficit symptoms compared with the model group.

### 2. The effect of the test substance on the cerebral infarction area

As shown in Table 9-1, the PEG conjugate of the mutant resulted in significant improvement in cerebral infarct size compared to the model group.

The combination of neurological deficit symptom score and cerebral infarct size results showed that after modified with the same PEG modifier, lysyl bradykinin mutants was more effective than lysyl bradykinin.

**Table 9-1 Effect of test subjects on neurological deficit symptoms and cerebral infarct size (%)**

| Groups | Total animal | Death | Sample | Neurological deficit symptom score | Cerebral infarct size (%) |
|---|---|---|---|---|---|
| Model group | 22 | 4 | 18 | 2.12 ± 0.17 | 29.23 ± 4.32 |
| PEG conjugates of lysyl bradykinin | 23 | 3 | 20 | 1.60 ± 0.17* | 22.82 ± 2.56 |
| PEG conjugates of lysyl bradykinin mutant | 23 | 3 | 20 | 1.50 ± 0.20* | 18.30 ± 1.08* |

| | | | | | |
|---|---|---|---|---|---|
| Mean ± SE. *P<0.05, compared with model group. | | | | | |

Considering the data from Examples 7 to 10, both PEG conjugates of lysyl bradykinin and PEG conjugates of different variants showed significant efficacy. PEG conjugates of Lys-BK, Lys-BKm01, Lys-BKm02, Lys-BKm11, Lys-BKm12, Lys-BKm13, Lys-BKm15 and Lys-BKm16 variants significantly improved neurological deficit symptoms and cerebral infarction size compared with the model group. There was statistical difference.

Lysyl bradykinin/bradykinin variants modified by PEG modifiers with different molecular weight (2K, 5K, 10K) showed good efficacy. Examples showed *in vivo* activity results of lysyl bradykinin/bradykinin variants modified with SPA modifiers, and in fact after modified with other types of polyethylene glycol modifiers, lysyl bradykinin/bradykinin or its variants retaining receptor binding activity also had significantly prolonged half-life *in vivo* and showed better activity *in vivo.*

Instead, lysyl bradykinin/bradykinin or its variants (wild type lysyl bradykinin/bradykinin, m01, m02, m11, m12, etc.) with no PEG modification or lysyl bradykinin/bradykinin variants with no receptor binding activity and PEG modification showed no improvement in neurological deficits in animal models at different doses and administration routes, maybe related to short half-life *in vivo.*

Of course, there are some differences in the efficacy of PEG conjugates of different lysyl bradykinin variants. Among them, the PEG conjugate of Lys-BKm13 variant was the most effective, and the Lys-BKm13 group had a extremely statistically significant improvement in neurological deficit symptoms and cerebral infarct size compared with the model group (P < 0.001) (Table 7-4). Lys-BKm11 had significant differences in the improvement of neurological deficit symptoms and cerebral infarction area compared with the model group (P < 0.01) (Table 7-3). Lys-BKm13 and Lys-BKm11 had only the first amino acid difference, indicating that the first Cys mutation had significant advantage to efficacy *in vivo.*

The difference between Lys-BKm11 and Lys-BKm01 was that Arg was inserted between the first and second amino acid of Lys-BKm01 to form Lys-BKm11, and Lys-BKm11 had certain advantages in improving the cerebral infarction area (Table 7-3).This conclusion was further confirmed by the comparison of Lys-BKm13 and Lys-BKm15, which showed that the PEG conjugate of Lys-BKm13 had better efficacy in the same condition (Table 7-6). Furthermore, additional Arg was inserted into Lys-BKm13 to obtain Lys-BKm16, and the efficacy of its PEG conjugate was equivalent to that of Lys-BKm13 (Table 7-5), indicating that the N-terminal insertion with Arg was more beneficial to the efficacy of polypeptide PEG conjugate.

The difference between Lys-BKm01 and Lys-BK was that unnatural amino acid was introduced, and the PEG conjugate of Lys-BKm01 had better efficacy than the PEG conjugate of Lys-BK (Table 9-1).

The C-terminus of Lys-BKm01 was lengthened to form Lys-BKm06. At the dose of 3mg/kg, the PEG conjugate of Lys-BKm06 had a tendency to improve neurological deficits and cerebral infarction size, but there was no significant difference compared with the model group, and its *in vivo* efficacy was lower than that of Lys-BKm01(Table 7-2). It indicated that the C-terminal structure was essential for the efficacy, and this result was consistent with the minimum active unit determination in Example 3. When the dose of PEG conjugate of Lys-BKm06 was further increased, the neurological deficit symptoms and cerebral infarction area were significantly improved.

In summary, the application has the following advantages over the prior art:
First, through the polyethylene glycol modification technology, the application greatly extends the half-life of lysyl bradykinin or bradykinin *in vivo,* and at the same time exerts its biological activity well. It solves the problem that lysyl bradykinin or bradykinin has very short half-life and cannot be used as drug. In terms of *in vivo* efficacy, PEG modified lysyl bradykinin or bradykinin or variants can significantly improve the neurological deficit symptom and cerebral infarction size in the cerebral ischemia-reperfusion model of MCAO.

Second, the modified variants of lysyl bradykinin or bradykinin in this application have obvious advantages, showing better stability in serum than the wild-type peptide, indicating prolonged half-life *in vivo,* while maintaining receptor affinity. Moreover, the PEG-modified variants showed better *in vivo* efficacy than PEG-modified wild-type lysyl bradykinin or bradykinin.

Third, the proposed PEG modified lysyl bradykinin or bradykinin show significant efficacy when administered intravenously, subcutaneously, or orally. In particular, subcutaneous injection and oral administration is convenient for clinical use. Patients do not need to go to the hospital regularly for injection, and they can administer drugs at home, which effectively improve the patient compliance and is more suitable for long-term treatment, such as recovery treatment of stroke patients.

## Claims

1. Polyethylene glycol modified lysyl bradykinin or bradykinin, wherein the PEG modification is N-terminal modification but not C-terminal modification, the lysyl bradykinin or bradykinin is wild-type lysyl bradykinin or bradykinin, or derivative derived from the wild-type lysyl bradykinin or bradykinin.

2. The polyethylene glycol modified lysyl bradykinin or bradykinin of claim 1, the PEG modifier used including but not limited to SPA, SCM, and SS modifiers.

3. The polyethylene glycol modified lysyl bradykinin or bradykinin of claim 1, the PEG modifier used may be linear or branched PEG modifier.

4. The polyethylene glycol modified lysyl bradykinin or bradykinin of claim 3, the PEG modifier used is straight-chain PEG modifier.

5. The polyethylene glycol modified lysyl bradykinin or bradykinin of claim 1, the molecular weight of PEG modifier used is 2KD-20KD.

6. The polyethylene glycol modified lysyl bradykinin or bradykinin of claim 1, the molecular weight of PEG modifier used is 2KD or 10KD.

7. Derivative of lysyl bradykinin or bradykinin, introducing cysteine at any position of the wild-type lysyl bradykinin or bradykinin sequence.

8. The derivative of lysyl bradykinin or bradykinin of claim 7, mutating the first Lys of wild-type lysyl bradykinin to cysteine.

9. The derivative of lysyl bradykinin or bradykinin of claim 7, which is inserted with any number and any kind of amino acid between the first and second amino acid residue at the N-terminus of wild-type lysyl bradykinin;or inserted with any number and any kind of amino acid before the first amino acid residue at the N-terminus of wild-type bradykinin.

10. The derivative of lysyl bradykinin or bradykinin of claim 7, wherein one or two or three amino acids are inserted between the first and second amino acid residues at the N-terminus of wild-type lysyl bradykinin; one or two or three amino acids are inserted before the first amino acid residue at the N-terminus of wild-type bradykinin.

11. The derivative of lysyl bradykinin or bradykinin of claim 9 or 10 , the inserted amino acid is arginine.

12. The derivative of lysyl bradykinin or bradykinin of claim 7, consists of two or more lysyl bradykinin monomers in series, or consists of two or more bradykinin monomers in series, or consists of lysyl bradykinin monomer and bradykinin monomer in series, said lysyl bradykinin or bradykinin monomer may be wild-type lysyl bradykinin or bradykinin, and also may be variant obtained by substitution, insertion, or deletion of some amino acids of wild-type lysyl bradykinin or bradykinin.

13. The derivative of lysyl bradykinin or bradykinin of claim 7, in which Phe at position 6 of wild-type lysyl bradykinin or Phe at position 5 of wild-type bradykinin is mutated to any other amino acid.

14. The derivative of lysyl bradykinin or bradykinin of claim 7 , the sequence of which is shown as SEQ ID NO: 14.

15. The polyethylene glycol modified lysyl bradykinin or bradykinin of any one of claims 1-6, said lysyl bradykinin or bradykinin is derivative of lysyl bradykinin or bradykinin of any one of claims 7-14.

16. Application of polyethylene glycol modified lysyl bradykinin or bradykinin of any one of claims 1-6, 15 in the preparation of drug for the treatment, prevention, recovery, and prevention of recurrence of ischemic stroke.

17. The polyethylene glycol modified lysyl bradykinin or bradykinin of any one of claims 1-6, 15 , which is administered by injection or orally.

18. Application of derivative of lysyl bradykinin or bradykinin of any one of claims 7-13 in the preparation of drug for the treatment, prevention, recovery, and prevention of recurrence of ischemic stroke.

19. Derivative of lysyl bradykinin or bradykinin of any one of claims 7-13, which is administered by injection or orally.
